# EUROPEAN PATENT APPLICATION

(11) **EP 1 743 624 A1**
(43) Date of publication of application: **17.01.2007**
(21) Application number: 05291507.1
(22) Date of filing: 12.07.2005
(51) Int. Cl.: A61K 8/67, A61K 8/06, A61K 8/89, A61K 8/891, A61Q 1/00, A61Q 19/00, A61K 8/892

(54) **Silicone-in-water emulsion compositions containing retinoids**

(71) Applicant: Johnson & Johnson Consumer France SAS, 92787 Issy les Moulineaux Cedex 9 (FR)
(72) Inventor: Brillouet, Anne Sophie, 27400 Louviers (FR); Gohier, Annie, 27310 Trinité (FR); Fournier, Linda, 27340 Pont de L' Arche (FR)
(74) Representative: Metten, Karl-Heinz

(57) **Abstract**

The present invention relates to a composition containing a retinoid wherein the composition is a silicone-in-water emulsion, and the use thereof.

## Description

### BACKGROUND OF THE INVENTION

An emulsion is generally a two-phase system that is prepared by combining two immiscible liquids, one of which is dispersed throughout the other (e.g., in the form of globules). The mixture is generally prevented from separating into distinct phases by the addition of an emulsifying agent or emulsifier. Emulsions are considered desirable as topical preparations due to their aesthetically pleasing appearance.

Retinoids generally have affinity with oils and waxes having a similar polarity as the retinoid. Current oil-in-water emulsions compositions that contain retinoids typically contains from about 20 to about 25 percent, by weight, of an oil phase, which is generally composed of a mixture of oils, waxes, and antioxidants in order to stabilize the retinoid, such as retinol. However, this high level of oils and/or waxes often result in products that generally feel oily or greasy on the skin.

Applicants have found that decreasing the ratio of the oil phase to the water phase in an emulsion in order to decrease the greasy feel of the product often results in a loss of stability of the retinoid in the composition, and thus a loss of efficacy.

The present invention relates to the discovery that silicone-in-water emulsions are unexpectedly effective for the topical administering retinoids, such as retinol. Such emulsions were unexpectedly found to enhance the stability of retinoids by having a high level of an internal oil phase, but yet still maintained strong consumer aesthetics. The silicone-in-water emulsions were also found to have superior efficacy, as the compositions were also unexpectedly found to have a high percentage of retinoid permeation.

### SUMMARY OF THE INVENTION

The present invention relates to a composition containing a retinoid wherein the composition is a silicone-in-water emulsion, and the use thereof.

Other features and advantages of the present invention will be apparent from the detailed description of the invention and from the claims.

### DETAILED DESCRIPTION OF THE INVENTION

It is believed that one skilled in the art can, based upon the description herein, utilize the present invention to its fullest extent. The following specific embodiments can be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Also, all publications, patent applications, patents, and other references mentioned herein are incorporated by reference. As used herein, all percentages are by weight unless otherwise specified.

### Definitions

As used herein, "topical application" and "topically applying" means directly laying on or spreading on the skin, hair, or nail, e.g., by use of the hands or an applicator such as a wipe.

As used herein, "cosmetically-acceptable" means that the retinoid, cosmetically active agents, inert ingredients, or composition which the term describes are suitable for use in contact with tissues (e.g., the skin) without undue toxicity, incompatibility, instability, irritation, allergic response, and the like, commensurate with a reasonable benefit/risk ratio.

As used herein, "safe and effective amount" means an amount of compound (e.g., the retinoid) or composition sufficient to significantly induce a positive modification in the condition to be regulated or treated, but low enough to avoid serious side effects. The safe and effective amount of the compound or composition will vary with the particular condition being treated, the age and physical condition of the end user, the severity of the condition being treated/prevented, the duration of the treatment, the nature of concurrent therapy, the specific compound or composition employed, the particular cosmetically-acceptable topical carrier utilized, and like factors.

### Silicone-in-Water Emulsion

Silicone-in-water emulsions according to the present invention includes two liquid phases, (i) a phase containing a liquid silicone and other lipophilic materials ("silicone phase" or "oil phase") and (ii) a phase containing water and other hydrophilic materials ("water phase" or "aqueous phase"). The silicone phase generally is from about 10 to about 50 percent by weight of the emulsion (such as from about 20 to about 40 percent by weight of the emulsion), and the water phase generally is from about 50 to about 90 percent by weight of the emulsion (such as from about 60 to about 80 percent by weight of the emulsion), based on the total weight of the emulsion.

The silicone phase includes a liquid silicone. Examples of liquid silicones include, but are not limited to, dimethicones and cyclomethicones (such as cyclotetrasiloxanes, cyclopentasiloxanes, cyclohexasiloxanes, and dimethyl cyclosiloxanes), phenyltrimethicones, caprylylmethicones, trisiloxane (and) dimethicone, cyclomethicone (and) dimethiconol, dimethicone (and) dimethiconol, and dimethicone crosspolymers. The one or more liquid silicones typically will be present in the composition in an amount from about 10% to about 45% by weight, in particular in an amount from about 20% to about 40% by weight.

In addition to the liquid silicone, the silicone phase may also contain one or more other non-silicone oils (such as mineral oil), thickeners, silicone-in-water emulsifiers, oil-soluble film-formers, oil-soluble antioxidants (such as tocopherols and BHT), humectants, oil-soluble anti-irritants (such as bisabolol), and silicone waxes (such as cetyl dimethicone).

Examples of thickeners include, but are not limited to, silicone thickeners such as dimethicone crosspolymers such as dimethicone/vinyl dimethicone crosspolymer and dimethicone/phenyl vinyl dimethicone crosspolymer. The thickeners typically will be present in the composition in an amount from about 0.001% to about 30% by weight, in particular in an amount from about 0.01% to about 12% by weight.

Examples of silicone-in-water emulsifiers include, but are not limited to, laureths (such as laureth-7, laureth-23, and laureth-4, and mixtures thereof), PEG- 12 Dimethicone, and dimethicone crosspolymer. The silicone-in-water emulsifiers typically will be present in the composition in an amount from about 0.001% to about 10% by weight, in particular in an amount from about 0.01% to about 5% by weight.

The water phase includes water. In addition to water, the water phase may also contain one or more water-soluble film-formers (such as high molecular weigh proteins such as hydrolyzed soy skin proteins), water-soluble thickeners (such as ammonium acryloyl dimethyl taurate/VP copolymer, sclerotium gum, C13-14 isoparaffin, and polyacrylamides), and water-soluble soothing agents (such as allantoin). Such water-soluble ingredients typically will be present in the composition in an amount from about 0.001% to about 10% by weight, in particular in an amount from about 0.01% to about 5% by weight.

### Retinoids

In one embodiment, the compositions of the present invention contain one or more retinoids. Examples of retinoids include, but are not limited to, retinol, retinal, retinoic acid, etretinate, acitretin, adapalene, tazarotene, and alitretinoin, and salts and esters thereof, such a retinyl palmitate and retinyl acetate.

In one embodiment, the composition comprises a safe and effective amount of the retinoid. The retinoid typically will be present in the composition in an amount from about 0.001% to about 10% by weight, in particular in an amount from about 0.01% to about 1% by weight.

### Additional Cosmetically Active Agents

In one embodiment, the topical composition further comprises another cosmetically active agent in addition to the retinoid. What is meant by a "cosmetically active agent" is a compound that has a cosmetic or therapeutic effect on the skin, hair, or nails, e.g., lightening agents, darkening agents such as self-tanning agents, anti-acne agents, shine control agents, anti-microbial agents, anti-inflammatory agents, anti-mycotic agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, firming agents, anti-callous agents, and agents for hair, nail, and/or skin conditioning.

In one embodiment, the cosmetically active agent is selected from, but not limited to, the group consisting of hydroxy acids, benzoyl peroxide, sulfur resorcinol, ascorbic acid, D-panthenol, hydroquinone, octyl methoxycinnimate, titanium dioxide, octyl salicylate, homosalate, avobenzone, polyphenolics, carotenoids, free radical scavengers, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, coenzyme Q10, lipoic acid, amino acids such a proline and tyrosine, vitamins, lactobionic acid, acetyl-coenzyme A, niacin, riboflavin, thiamin, ribose, electron transporters such as NADH and FADH2, and other botanical extracts such as aloe vera, feverfew, and soy, and derivatives and mixtures thereof. The cosmetically active agent will typically be present in the composition of the invention in an amount of from about 0.001% to about 20% by weight of the composition, e.g., about 0.01% to about 10% such as about 0.1% to about 5%.

Examples of vitamins include, but are not limited to, vitamin A, vitamin Bs (such as vitamin B3, vitamin B5, and vitamin B12), vitamin C, vitamin K, and vitamin E, and derivatives thereof.

Examples of hydroxy acids include, but are not limited, to glycolic acid, lactic acid, malic acid, salicylic acid, citric acid, and tartaric acid.

In one embodiment, the composition contains an antioxidant. Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, ascorbic acid, and ascorbic acid derivatives (e.g., ascorbyl palmitate and ascorbyl polypeptide). Oil-soluble antioxidants suitable for use in the compositions of this invention include, but are not limited to, butylated hydroxytoluene, tocopherols (e.g., tocopheryl acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis. Other examples of antioxidants may be found on pages 1612-13 of the ICI Handbook.

### Other Materials

Various other cosmetically-active agents may also be present in the skin care products. These include, but are not limited to, skin protectants, humectants, and emollients. The skin care products may also comprise chelating agents (e.g., EDTA), preservatives (e.g., parabens), pigments, dyes, opacifiers (e.g., titanium dioxide), and fragrances.

### Products

The silicone-in-water compositions of the present invention can be used for topical application to skin, hair, and nails. The compositions may be made into a wide variety of product types that include but are not limited to serums, lotions, creams, and make-up (such as foundations, mascaras, and lipsticks) and can thus be used for the manufacture of the aforementioned products, in particular cosmetic products.

In one embodiment, the composition and/or product is topically applied to the skin (e.g., the skin of a human in need of such treatment) for use in reducing the appearance of wrinkles on the skin (such as crow's feet, fine lines, and deep wrinkles), reducing the appearance of dark under-eye circles, treating acne, treating photodamage, reducing the appearance of hyperpigmentation such as age spots, reducing the appearance of roughness, sallowness, or pores on the skin. The composition may be applied by or be incorporated into, a wipe (such as a non-woven substrate) or a sponge pad.

The composition and products containing such compositions of the present invention may be prepared using methodology that is well known by an artisan of ordinary skill.

### Example 1: Compositions

The following are two examples of silicone-in-water emulsions of the present invention containing retinol (Examples 1A and 1B). The ingredients and their functions and corresponding weight percentage ranges are set forth in Table 1.

The water phase pre-mix ingredients were combined and dissolved in a pre-mix container.

In a primary container, the primary container water-phase ingredients were combined. These ingredients were mixed until homogeneous, and then the mixture in the pre-mix container was mixed with the mixture in the primary container.

The third container oil phase ingredients were combined in a third container. These ingredients were mixed until completely dissolved, and added to the primary container.

The post-add ingredients were then added and mixed in the primary container.

**Table 1**

| | **TRADENAME** | **INCI NAME** | **WEIGHT %** | |
|---|---|---|---|---|
| | | | **A** | **B** |
| PRIMARY CONTAINER | WATER, DEMINERALIZED | Water | Qs 100% | Qs 100% |
| | AMIGEL | Sclerotium Gum | 0.1 - 1 | 0.1 - 1 |
| | STRUCTURE XL | Hydroxypropyl Starch phosphate/Aqua | -- | 1 - 10 |
| | ARISTOFLEX AVC | Ammonium Acryloyldimethyl taurate/VP copolymer/Water | 0.1 - 3 | -- |
| | GLYCERIN 99.5% | Glycerin | 0.1 - 10 | 0.1 - 10 |
| | DISODIUM EDTA | Disodium EDTA | 0.01 - 0.1 | 0.01 - 0.1 |
| | ETHYLPARABEN | Ethylparaben | 0.05 - 0.3 | -- |
| | PHENOXYETHANOL | Phenoxyethanol | 0.1 - 1 | 0.1 - 1 |
| | METHYLPARABEN | Methylparaben | 0.05 - 0.3 | 0.05 - 0.3 |
| | PROPYLPARABEN | Propylparaben | 0.05 - 0.3 | 0.05 - 0.3 |
| | ALLANTOIN | Allantoin | 0.01 - 0.1 | -- |
| | SEPIGEL 305 | C13-14 Isoparaffin/ Laureth-7/ Polyacrylamide | 0.1 - 3 | -- |
| PRE-MIX CONTAINER | N,N DIMETHYLAMINE | DimethylMEA | 0.1 - 2.5 | 0.1 - 2.5 |
| | CITRIC ACID ANHYDROUS | Citric Acid | 0.1 - 2.5 | 0.1 - 2.5 |
| | WATER, DEMINERALIZED | Aqua | 5 - 10 | 5 - 10 |
| THIRD CONTAINER | FRAGRANCE | Perfume | 0.1 - 0.5 | 0.1 - 0.5 |
| | DC5329 PERFORMANCE MODIFIER | Dimethyl, Methyl (propyl (polyEO, PO) Hydroxy) siloxane, trimethylsiloxane-terminated;associated with PEG-12 Dimethicone/ Poly(ethylene oxide, propylene oxide) monoallylether/ Isopropyl Alcohol/ Cyclotetrasiloxane/ Propylene oxyde polymer with ethylene glycol | 0.1 - 3 | -- |
| | CYCLOPENTASILOXANE | Cyclopentasiloxane | 0.1 - 3 | 0.1 - 3 |
| | BHT | BHT | 0.01 - 0.1 | 0.01 - 0.1 |
| | ALPHA BISABOLOL NATUREL | Alpha Bisabolol | 0.1 - 0.5 | - - |
| | ORGASOL 2002EXD NAT COS | Nylon -12 | 0.1 - 3 | - - |
| | COVI-OX T90 EU | Tocopherols/ Helianthus Annuus Seed Oil | 0.1 - 3 | 0.1 - 3 |
| POST-ADD INGREDIENTS | DC 7-3101 EBPF HIP EMULSION | Cyclopentasiloxane/ Dimethicone Crosspolymer/ Dimethicone/ Dimethylcyclosiloxanes/ Water/ Cyclotetrasiloxanes/ Laureth-23/ Laureth-4 | 1 - 35 | - - |
| | DC 7-3118 EBPF HIP EMULSION | Cyclopentasiloxane/ Dimethicone Crosspolymer/ Dimethicone/ Dimethylcyclosiloxanes/ Water/ Cyclotetrasiloxanes/ Laureth-23/ Laureth-4 | - - | 1 - 35 |
| | POLYMERE DE BLE | Water/ Hydrolysed Wheat Polymer/ Sodium Methylparaben/ Sodium Ethylparaben/ Sodium Propylparben | 0.1 - 5 | 0.1 - 5 |
| | SEPITONIC M3 | Water/ Copper Gluconate/ Magnesium Aspartate/ Phenoxyethanol/ Zinc Gluconate | 0.1 - 2 | 0.1 - 2 |
| | ASORBIC ACID CRYSTALLINE | Ascorbic Acid | 0.01 - 0.5 | 0.01 - 0.5 |
| | RETINOL 50C | Retinol/ polysorbate 20/ BHT/ BHA | 0.01 - 0.3 | 0.01 - 0.3 |
| | COLORANT | Colorant | 0.001 - 0.01 | - - |
| | KSP 100 | Vinyl Dimethicone/ Methicone Silsequioxane | - - | 0.01 - 5 |
| | WATER, DEMINERALIZED | Water | 0.1 - 1 | - - |

### Example 2: Retinol Permeation

The permeation of retinol from a silicone-in-oil formulation of Example 1B was compared in an in-vitro model study with a commercially available oil-in-water emulsion containing approximately the same level of retinol to study the percutaneous penetration and absorption of retinol for a 24 hour period. In the test, pig skin was washed and the thickness of the skin was measured prior to mounting between the halves of vertical Franz-type diffusion cells (with the stratum corneum facing the donor chamber). The area of diffusion was about 2.54 cm². The diffusion cells were immersed in a bath maintained at 32°C and the chambers were filled with a PBS solution containing polyethylene glycol 20 oleyl ether and BHT. The products were then weighed and applied to the surface of the skin samples. After 24 hours (in the dark), the formulation remaining on the skin surface and the retinol that has penetrated into the skin was analyzed and quantified by HPLC.

As shown in Table 2 below, the efficiency of retinol permeation into the skin was unexpectedly increased by the silicone-in-water emulsion formulations of Example 1 as compared to the commercially available oil-in-water emulsion.

**Table 2**

| **TEST PRODUCT** | **Retinol Permeation** |
|---|---|
| Oil-in-water Product | 5% |
| Example 1B | 16% |

### Example 3: Stability

The stability of retinol in the silicone-in-water emulsion of Example 1B was compared to that of the oil-in-water emulsion composition containing approximately the same level of retinol, using HPLC with UV detection. Following six weeks under accelerated stability conditions at 50°C, only 1.5% of the retinol degraded, as compared to 65% of the retinol in the oil-in-water emulsion composition.

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A composition comprising a retinoid when said composition is a silicone-in-water emulsion.

2. A composition of claim 1, wherein said retinoid is retinol.

3. A composition of any of the preceding claims, wherein said composition comprises at least 50%, by weight, of a water phase.

4. A composition of any of the preceding claims, wherein said composition comprises at least 20%, by weight, of a silicone phase.

5. A composition of any of the preceding claims, wherein said composition comprises at least 10%, by weight, of one or more liquid silicones.

6. A composition of any of the preceding claims, wherein said composition comprises at least 20%, by weight, of one or more liquid silicones.

7. A composition of any of the preceding claims, wherein said composition comprises at least 10%, by weight, of a cyclomethicone.

8. A composition of any of the preceding claims, wherein said composition comprises a laureth.

9. A composition of any of the preceding claims, wherein the silicone phase of said emulsion comprises a dimethicone crosspolymer.

10. Use of a composition according to anyone of the preceding claims for the preparation of a cosmetic article to be used for the topical application to skin, hair or nails.
